# EUROPEAN PATENT APPLICATION

(11) **EP 3 928 783 A1**
(43) Date of publication of application: **29.12.2021**
(21) Application number: 20182108.9
(22) Date of filing: 24.06.2020
(51) Int. Cl.: A61K 38/08, A61K 39/12, A61P 31/14

(54) **MEDICAMENT FOR TREATMENT OF COVID-19**

(71) Applicant: Unthreadt B.V., 1086 ZP Amsterdam (NL)
(72) Inventor: Khan, Nisar Ahmed, 1086 ZP Amsterdam (NL); Benner, Robbert, 2992 SH Barendrecht (NL)
(74) Representative: Wittop Koning, Tom Hugo

(57) **Abstract**

The invention relates to a medicament for treatment of COVID-19 and related pathologies, the medicament comprising or interacting with one or more conserved regions of at least 4 consecutive amino acids with a 100% match present in both the SARS-CoV-2 proteome and the human proteome, wherein the one or more conserved regions are preferably selected by: a. identification of one or more conserved regions of at least 4 consecutive amino acids with a 100% match between the SARS-CoV-2 proteome and the human proteome; b. identification of at least one pathway class from a systematic database comprising a plurality of human physiological pathway classes, each class comprising a plurality of human proteins that are functionally related to the said physiological pathway, wherein the said at least one pathway class shares at least one pathology and/or complication of the COVID-19 infection as a result of dysfunction in the said pathway and comprises at least one human protein comprising one or more conserved regions of at least 4 consecutive amino acids that have a 100% match with the SARS-CoV-2 proteome; and c. selecting the said identified one or more conserved regions for the preparation of the medicament. Similarly, the invention also relates to a vaccine comprising one or more agents interacting with at least one region of at least 4 consecutive amino acids present in the SARS-CoV-2 proteome, not conserved between the SARS-CoV-2 proteome and the human proteome.

## Description

The invention relates to a medicament for treatment of COVID-19 and related pathologies.

Humans are suffering from outbreaks of Severe acute respiratory syndrome coronavirus (SARS-CoV) and Middle East respiratory syndrome coronavirus (MERS-CoV). A present pandemic viral outbreak is caused by Severe acute respiratory syndrome coronavirus-2 (SARS-CoV-2), which is a newly identified virus that differs from SARS-CoV and MERS-CoV but can cause largely similar symptomology associated with pneumonia. This viral disease was named "COVID-19" by the World Health Organization (WHO). The virus has spread to more than 200 countries and territories. WHO declared this disease to be a public health emergency of international concern. So far, there is no specific vaccine or effective treatment for MERS-CoV, SARS-CoV or SARS-CoV-2.

Viral infection involves a large number of genomic and protein interactions, including protein-protein interactions between the virus and its host. These interactions range from the initial binding of viral coat proteins to host membrane receptors to the hijacking of the host transcription machinery by viral proteins.

In current methods to identify and prepare a medicine against a microbial pathogen, great efforts are made to understand the life cycle of the pathogen and the mode of interaction with the host. Modern medicine focuses on disturbing the interaction mechanism of the pathogen, or to provide an immunological response to the pathogen by identifying epitopes that can be recognized by the host e.g. with the aim to provide a vaccine.

The present inventors have now found a completely novel and surprising approach as to identify new medicaments against SARS-CoV-2, i.e. for treatment of COVID-19. The new medicaments could be developed by the contemplation that important and decisive pathogen epitopes playing a role in infectious diseases may also be present in proteins that play an important role in one or more physiological pathways in the host. The rationale behind this is that the pathogen, in order to be specific for a particular host, would preferably use proteins or parts of proteins that are very similar to those of the host in order to interact with one or more biological pathways in the host. Such interaction may result in a particular pathology or complication in the host.

By this surprising approach, the inventors were capable to identify, by comparison of proteome data and physiological pathway analysis, a plurality of amino acid regions of at least 4 consecutive amino acids that can be used in the treatment of the infectious disease, in case COVID-19, or for treatment of related pathologies. The term 'related pathologies' refer to diseases that share one or more pathologies, complications or symptoms with those as brought about by the respective infection. The related diseases are not by infection by SARS-CoV-2, but are associated with similar pathologies, complications, or symptoms as those of a patient suffering from COVID-19. These pathologies, complications or symptoms can e.g. relate to breathing problems, such as acute respiratory distress syndrome (ARDS), or to those of a different viral infection.

The inventors were for the first time capable of identifying, on the one hand, important pathogenic epitopes that can be used to prepare a medicament against the said pathogen, whereas the said identification also enables the identification of endogenous proteins sharing such epitope sequences, which proteins play important roles in one or more physiological pathways that may be disturbed when suffering from a particular systemic disease. Such epitope or interaction therewith may therefore be useful in curing such a disease, *in casu* COVID-19.

The invention provides a method for treatment of COVID-19 and related pathologies, the said medicament comprising or interacting with at least 4 consecutive amino acids present with a 100% match in both the SARS-CoV-2 proteome and the human proteome.

The skilled person will be well capable of obtaining proteome information of the SARS-CoV-2 virus and of the human proteome. To this end, numerous databases and sequence information as well as useful software tools are available by which regions of at least 4 consecutive amino acids can be identified that are shared among one or more proteins present in human and one or more proteins encoded by the viral genome. The term 'conserved' for the amino acid region herein means that the amino acids region has a 100% identity, i.e. a 100% match between the pathogen and the host.

In an attractive embodiment, the one or more conserved regions of at least 4 consecutive amino acids with a 100% match are selected by:
a. identification of one or more conserved regions of at least 4 consecutive amino acids with a 100% match between the SARS-CoV-2 proteome and the human proteome;
b. identification of at least one pathway class from a systematic database comprising a plurality of human physiological pathway classes, each class comprising a plurality of human proteins that are functionally related to the said physiological pathway, wherein the said at least one pathway class shares at least one pathology and/or complication of the COVID-19 infection as a result of dysfunction in the said pathway and comprises at least one human protein comprising one or more conserved regions of at least 4 consecutive amino acids that have a 100% match with the SARS-CoV-2 proteome; and
c. selecting the said identified one or more conserved regions of at least 4 consecutive amino acids of step a., present in the at least one protein comprised in the pathway class identified in step b. for the preparation of the medicament.

In step a., conserved regions of at least 4 consecutive amino acids having a 100% match in both the human and the SARS-CoV-2 proteome are identified. Proteome data from both SARS-CoV-2 and human are known. Thereto, the proteome data from both the host and the pathogen are compared in order to identify the conserved region. The skilled person will be aware of suitable tools, that can e.g. be based on nucleic acid or amino acid sequence data.

Table 1 shows the distribution of peptide sequences shared by conserved peptide sequences of the SARS-CoV-2 proteome and the human proteome, and the occurrence in the various SARS-CoV-2 open reading frames. These data are based on the SARS-CoV-2 proteomes found in many different counties (number indicated).

**Table 1: occurrence of conserved regions of 4 - 7 amino acids**

| peptide length | distinct number of peptides | distinct number of ORF/proteins | from number of distinct countries |
|---|---|---|---|
| 4 | 2813 | 12 | 38 |
| 5 | 1146 | 12 | 38 |
| 6 | 136 | 11 | 38 |
| 7 | 11 | 5 | 33 |

Table 2 shows human peptide sequences that are identified that share a region of 5 consecutive amino acids with open reading frames of SARS-CoV-2.

**Table2: Human sequences with a 5-AA overlapping sequence with SARS-CoV-2.**

| **SARS-CoV-2** | **SARS-CoV-2 SEQ** | **Human peptide SEQ** |
|---|---|---|
| Nucleocapsid phosphoprotein | LLPAADLDD | AADLD, PAADL, LPAAD, AADLDD, LLPAADL |
| | SS(C/R)SSSRSR | SSCSS, SSRSR, SRSSS, SSSRSR, CSSSRS, CSSSRSR, SSCSSSR, SSRSSS |
| Surface glycoprotein | LVLLPLV | LLPLV, LVLLPL, VLLPL, VLLPLV |
| | GAGAAL | GAGAA, AGAAL |
| | VLPPLL | LPPLL, VLPPL, VLPPLL |
| ORF1ab | GGVAGA | GGVAG, GVAGA, GGVAG, GGVAGA |
| | AALGVL | ALGVL, AALGV, AALGVL |
| | DPAQLP | DPAQL, DPAQLP |
| | LLGVGG | LGVGG, LLGVG |
| | LGVLVP | LGVLV |
| | VLQAVGA | VLQAV, VLQAVG, LQAVG |
| | GVLPQLEQ | LPQLE, VLPQL, GVLPQ, PQLEQ |
| | VLQLPQG | QLPQG, LQLPQ, VLQLPQG |
| | ALPQRH | ALPQR, LPQRH |
| | ALQLEEE | LQLEE, ALQLE, ALQLEEE |
| | LPLQLG | LPLQL, LPLQLG |
| | VVADAV | VVADA,VVADAV |
| | RLALGG | LALGG, RLALGG |
| | LGGLHL | GGLHL, LGGLH, LGGLHL |
| ORF3a | ADGLEAP | ADGLE, DGLEA, GLEAP, ADGLEA |
| | QPEEEQE | EEEQE, PEEEQE, PEEEQ, PEEEQE, QPEEEQ, QPEEEQ |
| | LLLVA(A/V)(G/V)LE | AVGLE, LLVAV, LVAVG, VAVGL, VAVGLE, LLVAVG, LLVAAG, LLVAV, LLLVA, LVAAV, LVAAG |
| | VL(L/D)LLVA(A/V) | LLVAA, LLLVA, LDLLV, LLVAV, VLDLL, LLLLV |

In step b, a pathway class is identified from a systemic database that comprises a plurality of human physiological pathways. The term 'physiological pathway', herein also 'pathway' is well known in the art, and is defined herein as a network of chemical reactions by a group of proteins in a cell that work together to control one or more cell function(s). Gene, gene product or proteins are also classified based on their putative, predicted or experimentally confirmed role in one or more particular biological function(s) such as cellular component (the locations relative to cellular structures in which a gene product or protein performs a function, either cellular compartments, or stable macromolecular complexes of which they are parts), molecular function (molecular-level activities performed by gene product or protein) or biological process (the larger processes, or 'biological programs' accomplished by multiple molecular activities) (Ashburner et al. Gene ontology: tool for the unification of biology. Nat Genet. May 2000;25(1):25-9).

There are many pathways wherein proteins can be classified. For example, the KEGG database consists of about 2600 pathways, and the STRING database about 1800. Pathway classification databases can differ from one another in architecture and in definition of the classifications. Each of these databases discriminate a number of so-called 'top-level' pathways from 'lower level' pathways. Literature defines 27 top-level pathways (representing a broad area of biology) covering one or more levels of sub-pathways, becoming more specific with each lower level. These 27 top level pathways discriminated in mammalian molecular biology are: gene expression (transcription), cellular responses to external stimuli, DNA replication, disease, vesicle-mediated transport, extracellular matrix organization, haemostasis, transport of small molecules, chromatin organization, metabolism of proteins, developmental of biology, immune system, digestion and absorption, cell-cell communication, protein localization, programmed cell death, muscle contraction, reproduction, signal transduction, metabolism, neuronal system, autophagy, circadian clock, cell cycle, DNA repair, metabolism of RNA, and organelle biogenesis and maintenance. Accordingly, peptide sequences selected from the SARS-CoV-2 proteome can be related to any of the pathways, as defined in the respective database. Preferably, the envisaged one or more pathways are top-level pathways. In the SARS-CoV-2 proteome, some sequences can be identified that are most related to several 'top-level' pathways, while some other sequences are more specifically related to only some top-level pathways or a single top-level pathway. Based on the pathway or pathways whereto the said one or more proteins that comprise a conserved region of at least 4 consecutive amino acids belong. A medicament can be designed to cure a disease that is associated with dysfunction of the said pathway; such a disease is herein also defined as a pathway-associated disease. When the conserved region is present in one or more proteins present in a large number of different pathway classes, in particular top-level pathway classes, such a conserved region is a good candidate as active ingredient or part of an active ingredient against COVID-19.

Starting with step a., a comparison is made between the proteomic sequences of the pathogen, SARS-CoV-2 that are represented in the complete genome of the host. i.e. human. As a single continuous epitope is about 5 to 8 amino acids in length peptide sequences, peptide sequences of 5 to 7 amino acids can advantageously be analysed. The protein-protein interactions and pathway analyses tools (e.g. String database, KEGG database) involving these peptides identified 118 pathways, all with a small false discovery rate (FDR) of 0.05 or less. Pathways limited to those with an FDR cut-off of 10⁻⁴ are presented in Table 3. All these pathways are directly or indirectly associated with metabolism, longevity and biological aging or associated with known clinical manifestations and complications of COVID-19.

**Table 3: pathways selected from the whole human proteome on the basis of SARS-CoV-2 shared peptide sequences listed in Table 5.**

| | KEGG class PATH: | observed gene count | background gene count | false discovery rate |
|---|---|---|---|---|
| Insulin signalling pathway | ko04910 | 18 | 134 | 2.13E-21 |
| Longevity regulating pathway | ko04211 | 15 | 88 | 2.98E-19 |
| Insulin resistance | ko04931 | 15 | 107 | 2.86E-18 |
| Glutamatergic synapse | ko04724 | 15 | 112 | 4.02E-18 |
| Platelet activation | ko04611 | 15 | 123 | 1.17E-17 |
| Apelin signalling pathway | ko04371 | 15 | 133 | 2.87E-17 |
| Oxytocin signally pathway | ko04921 | 15 | 149 | 1.19E-16 |
| Circadian entrainment | ko04713 | 13 | 93 | 4.14E-16 |
| Calcium signalling pathway | ko04020 | 15 | 179 | 1.19E-15 |
| Taste transduction | ko04742 | 12 | 81 | 3.30E-15 |
| Gap junction | ko04540 | 12 | 87 | 6.55E-15 |
| Dilated cardiomyopathy (DCM) | ko05414 | 12 | 88 | 6.81 E-15 |
| Glucagon signalling pathway | ko04922 | 11 | 100 | 9.24E-13 |
| Adrenergic signally in cardiomyocytes | ko04261 | 11 | 139 | 2.46E-11 |
| Retrograde endocannabinoid signally | ko04723 | 11 | 148 | 4.37E-11 |
| Cholinergic synapse | ko04725 | 10 | 111 | 7.04E-11 |
| cGMP-PKG signally pathway | ko04022 | 11 | 160 | 8.60E-11 |
| AMPK signalling pathway | ko04152 | 10 | 120 | 1.30E-10 |
| Hypertrophic cardiomyopathy (HCM) | ko05410 | 9 | 81 | 1.30E-10 |
| Regulation of lipolysis in adipocytes | ko04923 | 8 | 53 | 1.85E-10 |
| Oestrogen signalling pathway | ko04915 | 10 | 133 | 2.90E-10 |
| Aldosterone synthesis and secretion | ko04925 | 9 | 93 | 3.45E-10 |
| Long-term depression | ko04730 | 8 | 60 | 4.00E-10 |
| cAMP signalling pathway | ko04024 | 11 | 195 | 4.65E-10 |
| Phospholipase D signalling pathway | ko04072 | 10 | 145 | 5.47E-10 |
| Serotonergic synapse | ko04072 | 9 | 112 | 1.38E-09 |
| Thermogenesis | ko04714 | 11 | 228 | 2.03E-09 |
| Vascular smooth muscle contraction | ko04270 | 9 | 119 | 2.13E-09 |
| Autophagy - animal | | 9 | 125 | 3.10E-09 |
| Salivary secretion | ko04970 | 8 | 86 | 4.36E-09 |
| Endocrine resistance | | 8 | 95 | 8.81 E-09 |
| Cortisol synthesis and secretion | hsa04927 | 7 | 63 | 1.58E-08 |
| Long-term potentiation | ko04720 | 7 | 64 | 1.70E-08 |
| Type II diabetes mellitus | hsa04930 | 6 | 46 | 8.81 E-08 |
| Proteoglycans in cancer | hsa05205 | 9 | 195 | 1.04E-07 |
| Morphine addiction | hsa05032 | 7 | 91 | 1.51 E-07 |
| Inflammatory mediator regulation of TRP channels | hsa04750 | 7 | 92 | 1.58E-07 |
| Pancreatic secretion | hsa04972 | 7 | 95 | 1.90E-07 |
| Renin secretion | hsa04924 | 6 | 63 | 4.34E-07 |
| Adipocytokine signalling pathway | hsa04920 | 6 | 69 | 7.01 E-07 |
| Gastric acid secretion | hsa04971 | 6 | 72 | 8.66E-07 |
| Arrhythmogenic right ventricular cardiomyopathy | | 6 | 72 | 8.66E-07 |
| Thyroid hormone synthesis | | 6 | 73 | 8.92E-07 |
| Cardiac muscle contraction | | 6 | 76 | 1.09E-06 |
| Dopaminergic synapse | | 7 | 128 | 1.11 E-06 |
| EGFR tyrosine kinase inhibitor resistance | | 6 | 78 | 1.20E-06 |
| Relaxin signalling pathway | | 7 | 130 | 1.20E-06 |
| Rap1 signalling pathway | | 8 | 203 | 1.56E-06 |
| Insulin secretion | | 6 | 84 | 1.71 E-06 |
| MAPK signalling pathway | | 9 | 293 | 2.07E-06 |
| GABAergic synapse | | 6 | 88 | 2.13E-06 |
| GnRH signalling pathway | | 6 | 88 | 2.13E-06 |
| Endocrine/other factor-regulated calcium reabsorption | | 5 | 47 | 2.38E-06 |
| Ovarian steroidogenesis | | 5 | 49 | 2.83E-06 |
| Cushing's syndrome | | 7 | 153 | 2.87E-06 |
| Chemokine signalling pathway | | 7 | 181 | 8.28E-06 |
| Thyroid hormone signalling pathway | | 6 | 115 | 8.40E-06 |
| Oocyte meiosis | | 6 | 116 | 8.66E-06 |
| Starch and sucrose metabolism | | 4 | 33 | 1.82E-05 |
| Pathways in cancer | | 10 | 515 | 2.21 E-05 |
| Human papillomavirus infection | | 8 | 317 | 3.04E-05 |
| Progesterone-mediated oocyte maturation | | 5 | 94 | 4.98E-05 |
| PI3K-Akt signalling pathway | | 8 | 348 | 5.68E-05 |
| Tight junction | | 6 | 167 | 5.82E-05 |
| Melanogenesis | | 5 | 98 | 5.82E-05 |
| Neuroactive ligand-receptor interaction | | 7 | 272 | 9.16E-05 |

E.g. from the KEGG database, additional information as to the pathway classes can be retrieved, such as other pathways that are related to the primary pathways shown in table 3. Also, the physiological effects of the pathways can be retrieved from the said database, based upon which pathway-related diseases can be identified by the skilled person.

From analysis of the first 40 pathways from Table 3 (with an FDR ranging from 2.13 x 10⁻²¹ to 8.66 x 10⁻⁷) and the pathways that according to the KEGG database are related to these first 40 pathways show that their effects are not only associated with the clinical manifestations and/or complications of COVID-19, but also are directly or indirectly related to the underlying cellular processes and their functional manifestations, as shown in table 4.

**Table 4: Pathway pathologies, related pathways, cellular processes and manifestations**

| Pathology/complications | Related pathways, underlying cellular processes and functional manifestations |
|---|---|
| Cardiovascular | Dilated cardiomyopathy (DCM), Insulin signalling pathway, Apelin signalling pathway, Longevity regulating pathway, Vascular smooth muscle contraction, Renin secretion, Arrhythmogenic right ventricular cardiomyopathy, Cardiac muscle contraction, Relaxin signalling pathway, Insulin secretion, mTOR signalling pathway, AMPK signalling pathway, Vasopressin-regulated water reabsorption |
| Neurological | Glutamatergic synapse, Cholinergic synapse, Long-term depression, Long-term potentiation, Dopaminergic synapse, GABAergic synapse, GnRH signalling pathway, Alzheimer's disease. |
| Gustatory | Taste transduction, Salivary secretion |
| Renal | Cortisol synthesis and secretion, Renin secretion, AMPK signalling pathway, mTOR signalling pathway, Circadian entrainment, Vasopressin-regulated water reabsorption |
| Temperature | Thermogenesis pathway |
| Inflammation | Insulin signalling pathway, Longevity regulating pathway, Platelet activation, cGMP-PKG signalling pathway, Oestrogen signalling pathway, Phospholipase D signalling pathway, Cortisol synthesis and secretion, Inflammatory mediator regulation of TRP channels, Rap1 signalling pathway, MAPK signalling pathway, Chemokine signalling pathway, PI3K-Akt signalling pathway, Sphingolipid signalling pathway, Fc gamma R-mediated phagocytosis, Natural killer cell mediated cytotoxicity, Necroptosis, Fc epsilon RI signalling pathway, Viral myocarditis, Autophagy - animal, Circadian entrainment |
| Cardiovascular | Dilated cardiomyopathy (DCM), Insulin signalling pathway, Apelin signalling pathway, Longevity regulating pathway, Vascular smooth muscle contraction, Renin secretion, Arrhythmogenic right ventricular cardiomyopathy, Cardiac muscle contraction, Relaxin signalling pathway, Insulin secretion, mTOR signalling pathway, AMPK signalling pathway, Vasopressin-regulated water reabsorption |
| BMI (obesity) | Insulin resistance, Insulin signalling pathway, Glucagon signalling pathway, AMPK signalling pathway, Endocrine resistance, Pancreatic secretion, Adipocytokine signalling pathway, Gastric acid secretion, Insulin secretion, Ovarian steroidogenesis, Circadian entrainment, Type II diabetes mellitus, Carbohydrate digestion and absorption |
| Psychiatry | Oxytocin signalling pathway, Circadian entrainment, Serotonergic synapse, Dopaminergic synapse, GABAergic synapse, GnRH signalling pathway, Long-term potentiation, Long-term depression |
| Addiction | Morphine addiction, Amphetamine addiction, Cocaine addiction, Alcoholism, Nicotine addiction |

It was found that SARS-CoV-2 shared peptide sequences in human proteins occur in several pathways, and other peptide sequences in fewer pathways or even in a single pathway. The peptides identified in this manner are listed in Table 5 in the column Shared SEQ. The values in the other columns represent the proportion of pathways that have one or more proteins with at least one shared peptide. The value zero indicates that that particular peptide does not show up in this analysis.

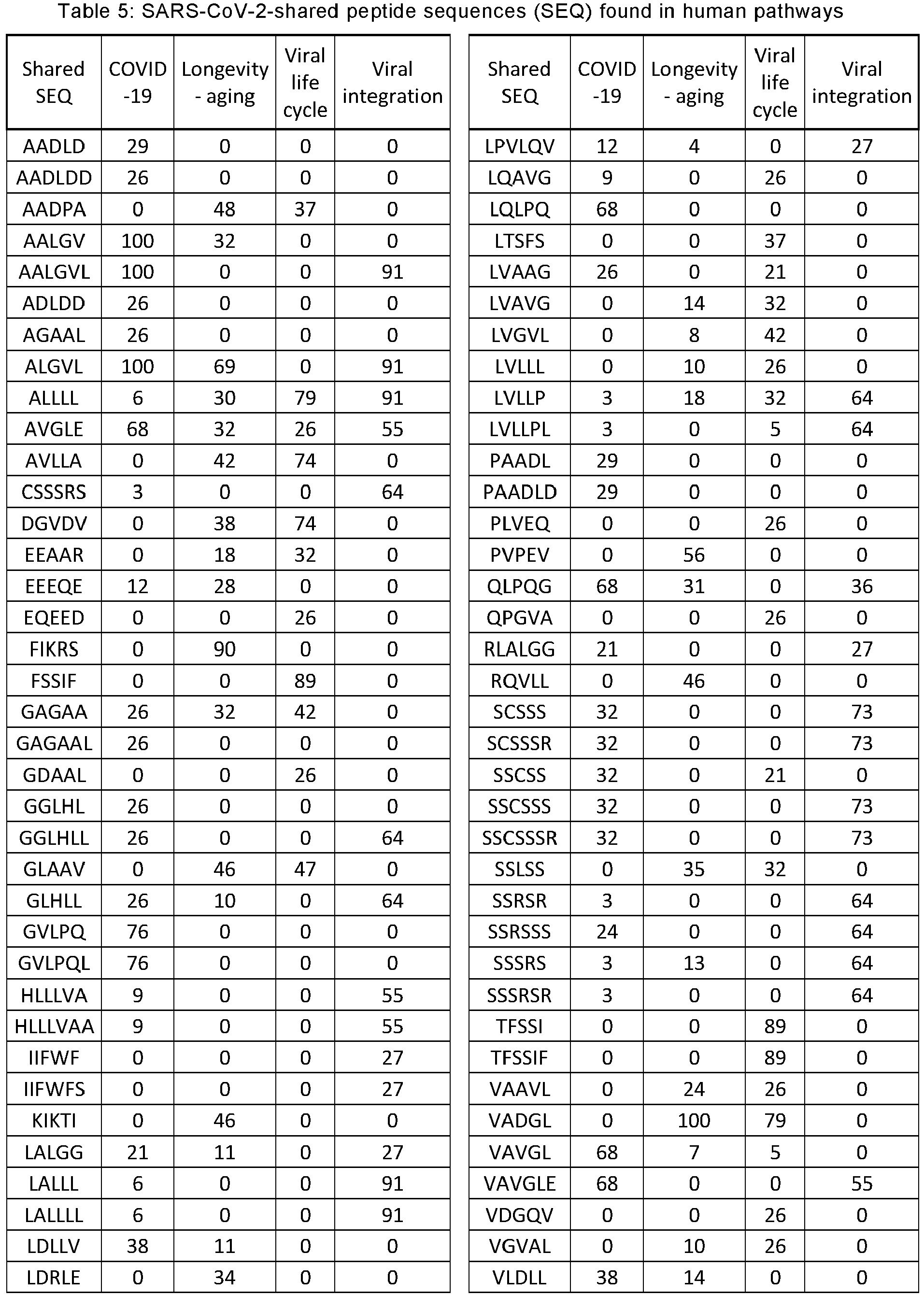

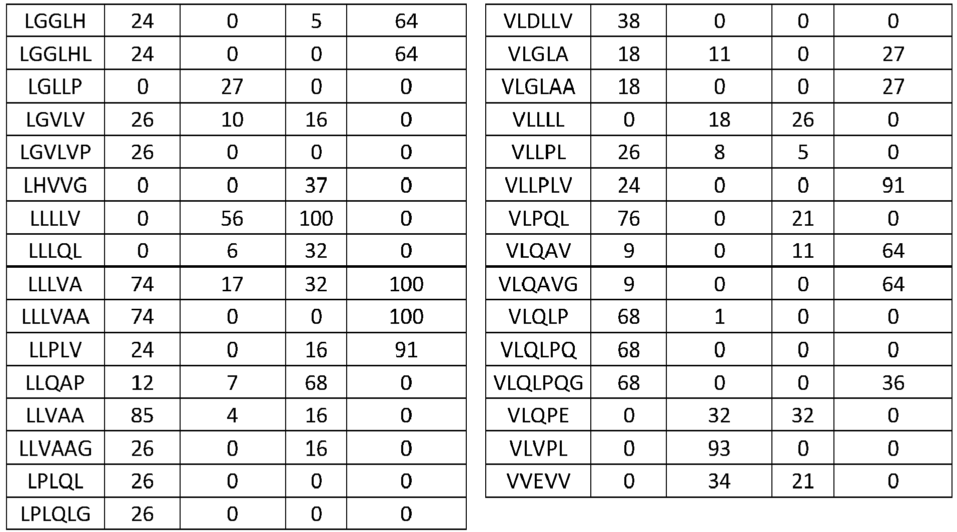

5-, 6- and 7-mer peptide sequences were identified that include a 5-mer sequence only, selected from proteins that are found to be directly or indirectly related with the pathways metabolism, longevity and biological aging as well as associated with known clinical manifestations and complications of COVID-19 (tables 3 and 4.).

A high significance was observed for the biological processes longevity and aging related pathway classes, as well as for the biological process viral life cycle related pathway class in COVID-19.

In an attractive embodiment of the invention, it is also possible to identify one or more biological processes of the host first, and then check for conserved regions of at least 4 amino acids present in both one or more proteins of the said biological class, and in the genome of the pathogen. Accordingly, the above step a. of identification of one or more conserved regions between the SARS-CoV-2 proteome and the human proteome is replaced by the following steps a1 and a2:

| | |
|---|---|
| step a1. | comprising identification of one or more biological processes from a systematic database, the database comprising a plurality of human biological processes and, for each biological process, a plurality of human proteins that are functionally related to the said biological process, and |
| step a2. | comprising identification of at least one conserved region of at least 4 consecutive amino acids in one or more of the plurality of proteins |
| | identified in step a1., said conserved region of at least 4 consecutive amino acids having a 100% match with the SARS-CoV-2 proteome. |

A biological process is defined as a series of molecular events, with a defined beginning and end; biological processes are assumed to be independent and do not represent the interactions among molecules, whereas a biological pathway is a network of chemical reactions, and one or more thereof can be part of the biological process (Gene Ontology, Nat. Genet. May 2000; 25(1): 25-29). Databases comprising a plurality of biological processes and a plurality of proteins that are categorized among the different process classes according to the functionality of the said proteins are known in the art, such as e.g. the gene Ontology Database (supra).

Accordingly, an inventory of one or more significant or otherwise envisaged classes of biological processes can be made e.g. in order to identify one or more regions of at least 4 consecutive amino acids present in the said one or more classes of biological process, in order to be used as an active ingredient in a medicament against an envisaged disease. In that case, an inventory of amino acid regions of at least 4 consecutive amino acids present in proteins belonging to the envisaged one or more classes of biological processes is made, followed by a comparison with the SARS-CoV-2 proteome, and identification of one or more biological pathways as described above. Said pathways are preferably identified among pathways, known to be involved in the said biological process. This way, the identification of conserved amino acids is preceded by a functional analysis instead of starting with a proteome comparison.

These above-mentioned biological processes of longevity and aging, viral life cycle, and viral integration were chosen for this approach, i.e. starting with these classes of biological classes as identified biological class. According to this approach, it is also possible to identify one or more subclasses, based on a first identification of a biological process, followed by identification of one or more conserved regions of at least 4, preferably at least 5 identical consecutive amino acids in proteins of the biological processes. Those conserved regions then serve as starting point for the identification of one or more pathway subclasses according to the above.

By identifying the biological process of longevity and ageing, 274 human Swiss-Prot proteins from the Gene Ontology database, related to the biological processes term 'aging' were retrieved. 191 proteins thereof contain at least one 5- and/or 6-mer that also occurs in the various SARS-CoV-2 proteins. 341 peptide sequences with one or more 5- and/or 6-mers were shared by the 191 human proteins involved in the 'aging' and SARS-CoV-2 proteins. The protein-protein interactions and pathways analyses tools (e.g. String database, KEGG database) involving these peptides identified 129 pathways, all with an FDR smaller than 0.05. Table 6 shows those with an FDR of less than 10⁻⁴.

The results show that some SARS-CoV-2 shared peptide sequences in human proteins occur in several pathways, and other peptide sequences in fewer pathways or even in a single pathway. The peptides identified in this manner are listed in Table 5 in the column Longevity/aging.

Dysfunctions associated with the most significant sub-pathway of AGE-RAGE signalling-mediated diabetic complications include diabetic neuropathy, diabetic nephropathy, diabetic vascular complications, and diabetic foot syndrome. In addition, AGEs also play an important role in most age-related diseases such as Alzheimer's, cancer, cardiovascular disease, kidney disease, high blood pressure, stroke, visual impairment, and skin diseases. In many cases of COVID-19, not only after intensive care treatment, patients suffer for months of memory problems, focusing problems, sleeping problems, gloom, fear and muscle weakness, all symptoms associated with ageing. These late effects may well be related to SARS-CoV-2 affected pathways shown in Table 3, e.g. oxytocin signalling, circadian entrainment, cholinergic synapse, long-term depression, and serotonergic synapse.

**Table 6: Human pathways selected on the basis of 5- and 6-mer SARS-CoV-2 sequences present in human proteins related to biological process aging and longevity based on sequences of table 5.**

| Pathways | count in gene set | false discovery rate |
|---|---|---|
| AGE-RAGE signalling pathway in diabetic complications | 14 of 98 | 8.64E-10 |
| FoxO signalling pathway | 15 of 130 | 1.16E-09 |
| Cellular senescence | 14 of 156 | 8.25E-08 |
| Apoptosis | 13 of 135 | 1.13E-07 |
| Pathways in cancer | 23 of 515 | 1.13E-07 |
| Longevity regulating pathway | 11 of 88 | 1.14E-07 |
| HTLV-I infection | 16 of 250 | 2.16E-07 |
| Pancreatic cancer | 10 of 74 | 2.33E-07 |
| Colorectal cancer | 10 of 85 | 6.91E-07 |
| Acute myeloid leukaemia | 9 of 66 | 9.95E-07 |
| HIF-1 signalling pathway | 10 of 98 | 1.95E-06 |
| Relaxin signalling pathway | 11 of 130 | 2.35E-06 |
| MAPK signalling pathway | 15 of 293 | 5.59E-06 |
| Neurotrophin signalling pathway | 10 of 116 | 6.56E-06 |
| Viral carcinogenesis | 12 of 183 | 6.62E-06 |
| MicroRNAs in cancer | 11 of 149 | 6.62E-06 |
| AMPK signalling pathway | 10 of 120 | 6.92E-06 |
| Central carbon metabolism in cancer | 8 of 65 | 6.92E-06 |
| Prolactin signalling pathway | 8 of 69 | 9.38E-06 |
| Chagas disease (American trypanosomiasis) | 9 of 101 | 1.38E-05 |
| Oestrogen signalling pathway | 10 of 133 | 1.41E-05 |
| Chronic myeloid leukaemia | 8 of 76 | 1.59E-05 |
| EGFR tyrosine kinase inhibitor resistance | 8 of 78 | 1.82E-05 |
| Insulin resistance | 9 of 107 | 1.82E-05 |
| TNF signalling pathway | 9 of 108 | 1.85E-05 |
| Hepatitis B | 10 of 142 | 1.98E-05 |
| Kaposi's sarcoma-associated herpesvirus infection | 11 of 183 | 2.52E-05 |
| Endometrial cancer | 7 of 58 | 2.52E-05 |
| Gastric cancer | 10 of 147 | 2.52E-05 |
| Longevity regulating pathway - multiple species | 7 of 61 | 3.19E-05 |
| Human papillomavirus infection | 14 of 317 | 3.19E-05 |
| Osteoclast differentiation | 9 of 124 | 4.19E-05 |
| cGMP-PKG signalling pathway | 10 of 160 | 4.26E-05 |
| Non-small cell lung cancer | 7 of 66 | 4.54E-05 |
| Hepatocellular carcinoma | 10 of 163 | 4.70E-05 |
| Glioma | 7 of 68 | 5.14E-05 |
| Prostate cancer | 8 of 97 | 5.14E-05 |
| Adipocytokine signalling pathway | 7 of 69 | 5.32E-05 |
| B cell receptor signalling pathway | 7 of 71 | 6.17E-05 |
| Cocaine addiction | 6 of 49 | 8.55E-05 |
| Chemokine signalling pathway | 10 of 181 | 9.54E-05 |

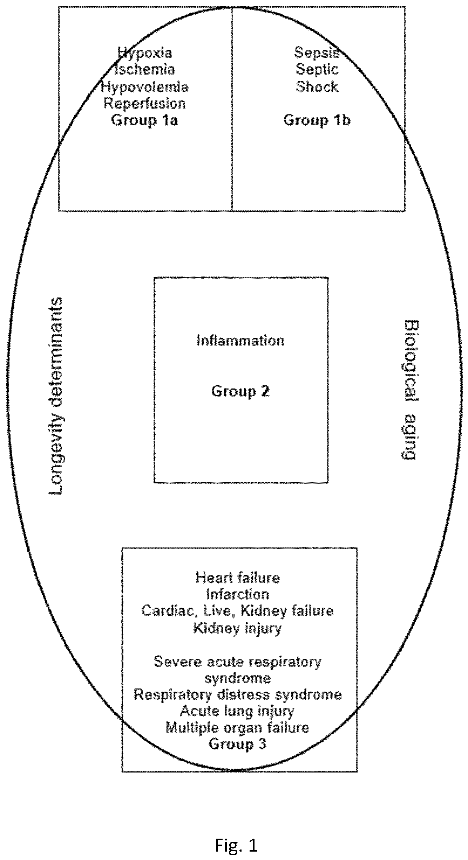

A simplified scheme of the sequence of (patho)physiological events leading to various pathological conditions of which the outcome is determined by the genes or their (by)products related to longevity pathways and aging is given in figure 1. Various endogenous and exogenous "insults", such as either non-microbes (group 1a), e.g. hypoxic, ischemic, hypovolemic or reperfusion events, or as microbes (group 1b), e.g. bacteria or virus in sepsis and septic shock, lead to inflammatory responses (group 2). These responses can be beneficial or detrimental to various organs (group 3). The outcome of these responses is dependent on the various determinants of longevity pathways and biological aging.

Starting from the biological process of viral life cycle, 210 human Swiss-Prot proteins were retrieved from Gene Ontology database, related to the biological processes term 'viral life cycle'. Proteins related to the viral life cycle are proteins that influence a set of processes which all viruses follow to ensure their multiplication and survival. These include attachment and entry of the virus particle, decoding of genomic information, translation of viral mRNA by host ribosomes, genome replication, and assembly and release of viral particles containing the genome. 137 of the said 210 proteins contain at least two 5- and/or 6-mers that also occur in the various SARS-CoV-2 proteins, see Table 7.

**Table 7: Number of distinct 5- and 6-mers 'conserved' peptide sequences from SARS-CoV-2 coded proteins that also occur at least twice in human proteins related to 'viral life cycle' biological process.**

| Sars-CoV-2 protein name | Number of distinct peptides |
|---|---|
| S surface glycoprotein | 30 |
| E envelope protein | 6 |
| M membrane protein | 5 |
| N nucleocapsid phosphoprotein | 32 |
| ORF1a | 122 |
| ORF1ab | 174 |
| ORF3a | 68 |
| ORF6 | 12 |
| ORF7a | 1 |
| ORF7b | 4 |
| ORF8 | 7 |

The protein-protein interactions and pathways analyses tools (e.g. String database, KEGG database) involving these peptides identified 39 pathways, all with an FDR smaller than 0.05. Table 8 shows those having an FDR of less than 10⁻⁴. The top 2 pathways of this list concern endocytosis with an FDR of 2.40x10⁻²² and RNA transport with an FDR of 8.08x10⁻¹⁵. The results show that some SARS-CoV-2 shared peptide sequences in human proteins occur in several pathways, and other peptide sequences in fewer pathways or even in a single pathway. The peptides identified in this manner are listed in Table 6 in the column Viral life cycle. The values in this column represent the proportion of pathways that have one or more proteins with at least one shared peptide. The value zero indicates that that particular peptide does not show up in this analysis.

The human proteins containing SARS-CoV-2 peptide sequences involved in the 'viral life cycle' appeared to be also involved in a number of other diseases and biological processes, see Table 8. These shared 5- and 6-mer peptide sequences can be synthesized according to known procedures and used not only as pharmaceuticals to modulate the biological processes involved in 'viral life cycle', but also to modulate several other related diseases and biological processes based on pathways mentioned in Table 8. A COVID-19 related pathology also encompasses other viral infections, that share one or more pathologies/complications.

**Table 8: Human pathways involved in the virus life cycle selected on the basis of the 5- and 6-mer peptide sequences listed in Table 5.**

| KEGG pathway | count in gene set | false discovery rate |
|---|---|---|
| Endocytosis | 27 of 242 | 2.40E-22 |
| RNA transport | 18 of 159 | 8.08E-15 |
| Cell adhesion molecules | 11 of 139 | 2.03E-07 |
| Phagosome | 11 of 145 | 2.30E-07 |
| Arrhythmogenic right ventricular cardiomyopathy | 8 of 72 | 1.52E-06 |
| Hypertrophic cardiomyopathy | 8 of 81 | 2.94E-06 |
| ECM-receptor interaction | 8 of 81 | 2.94E-06 |
| Focal adhesion | 11 of 197 | 2.94E-06 |
| Necroptosis | 10 of 155 | 2.94E-06 |
| Dilated cardiomyopathy (DCM) | 8 of 88 | 3.18E-06 |
| Hematopoietic cell lineage | 8 of 94 | 4.63E-06 |
| Proteoglycans in cancer | 9 of 195 | 9.62E-05 |

Starting from the pathway class of viral integration, conserved 5-, 6- and 7-mer SARS-CoV-2 peptide sequences that include a 5-mer sequence only, that were related to the viral integration into the host cell pathways were identified. According to KEGG and STRING databases, the related pathways are the following: RNA polymerase, RNA polymerase I (involved in chain elongation, promoter clearance, promotor escape, transcription, transcription initiation and transcription termination), RNA polymerase II (involved in pre-transcription events, transcription pre-initiation and promotor opening, transcription, transcription initiation and promotor clearance, transcription elongation, transcription termination, transcribes snRNA genes, HIV promotor escape, promotor escape), RNA polymerase III (involved in transcription, transcription initiation, transcription initiation from type 1, type 2 and type 3 promotor, abortive and retractive initiation, chain elongation, termination), Influenza viral RNA transcription and replication, host interactions with influenza factors, influenza infection, influenza life cycle, influenza A, Epstein-Barr virus infection, integration of provirus, influenza viral RNA transcription and replication, antiviral mechanism by IFN-stimulated genes, export of viral ribonucleoproteins from nucleus, ISG15 antiviral mechanism, viral messenger RNA synthesis, viral carcinogenesis, viral myocarditis and viral mRNA translation. The selected human sequences can be synthesized and used as pharmaceuticals to modulate the viral integration into the host cell.

Based on the above peptide sequences, related human proteins that contain these SARS-CoV-2 peptide sequences were identified and used in the KEGG database to identify the related pathways. Table 9 shows the 28 pathways identified on this basis.

The results show that some SARS-CoV-2 shared peptide sequences in human proteins occur in several pathways, and other peptide sequences in fewer pathways or even in a single pathway. The peptides identified in this manner are listed in Table 6 in the column Viral integration. The values in this column represent the proportion of pathways that have one or more proteins with at least one shared peptide. The value zero indicates that that particular peptide does not show up in this analysis.

**Table 9: Human pathways selected on the basis of SARS-CoV-2 peptide sequences related to the viral integration into the host cell pathways based on the sequence listed in table 5.**

| KEGG pathway | count in gene set | false discovery rate |
|---|---|---|
| Epstein-Barr virus infection | 35 of 194 | 6.27E-08 |
| Herpes simplex infection | 25 of 181 | 0.00092 |
| Viral carcinogenesis | 24 of 183 | 0.002 |
| HIF-1 signalling pathway | 16 of 98 | 0.0033 |
| Influenza A | 22 of 168 | 0.0033 |
| Apelin signalling pathway | 18 of 133 | 0.0073 |
| Measles | 18 of 133 | 0.0073 |
| Human papillomavirus infection | 31 of 317 | 0.0086 |
| Toxoplasmosis | 15 of 109 | 0.0143 |
| Pathways in cancer | 42 of 515 | 0.0222 |
| Spliceosome | 16 of 130 | 0.023 |
| FoxO signalling pathway | 16 of 130 | 0.023 |
| RNA transport | 18 of 159 | 0.0232 |
| Insulin signalling pathway | 16 of 134 | 0.0242 |
| Platelet activation | 15 of 123 | 0.0262 |
| Tight junction | 18 of 167 | 0.0311 |
| EGFR tyrosine kinase inhibitor resistance | 11 of 78 | 0.0342 |
| ABC transporters | 8 of 44 | 0.0342 |
| Hepatitis B | 16 of 142 | 0.0342 |
| Rap1 signalling pathway | 20 of 203 | 0.0375 |
| NF-kappa B signalling pathway | 12 of 93 | 0.0375 |
| Jak-STAT signalling pathway | 17 of 160 | 0.0375 |
| Endocrine resistance | 12 of 95 | 0.0382 |
| ErbB signalling pathway | 11 of 83 | 0.0385 |
| Adherens junction | 10 of 71 | 0.0385 |
| Osteoclast differentiation | 14 of 124 | 0.0407 |
| Ras signalling pathway | 21 of 228 | 0.0456 |
| Pancreatic cancer | 10 of 74 | 0.0456 |

All anti-viral peptide sequences identified herein can be synthesized according to known procedures and used for the anti-viral treatment of COVID-19 patients, and patients with other viral diseases. These pharmaceuticals will also be useful for the treatment of animals with a viral infection. Similar analyses can be done for any other biological process, molecular function and cellular component starting from the related human proteins listed in e.g. the Gene Ontology database.

It is to be understood that the above identification is a general concept, applicable to virtually all infectious diseases in any host from any pathogen. In step a., the proteome of the host and that of the pathogen are compared in order to identify conserved regions of at least 4 consecutive amino acids, and in step b., one or more pathway classes are identified from a systematic database as described above, comprising pathway classes and protein data of the host.

In accordance with the above, it is also possible to identify conserved regions of at least 4 consecutive amino acids in one or more proteins in the pathogen that are not shared by human proteins, or not shared by human proteins that play a role in one or more defined physiological pathways, preferably not shared by any human protein. Such a stretch of at least 4 consecutive amino acids can be used for the preparation of e.g. a vaccine. Therefore, in another embodiment, the medicament is a vaccine against COVID-19, the said vaccine comprising one or more agents interacting with at least one region of at least 4 consecutive amino acids present in the SARS-CoV-2 proteome, the said at least one region of at least 4 consecutive amino acids being selected by identification of regions of at least 4 consecutive amino acids not conserved between the SARS-CoV-2 proteome and the human proteome; and selecting the said region of at least 4 consecutive amino acids for the preparation of the vaccine. This novel approach for explaining the clinical manifestations and complications of COVID-19 and finding of peptides for their effective treatment can also be used for defining safe genomic and proteomic regions for the design of safe vaccines (e.g. SARS-CoV-2 vaccines) by avoiding conserved regions shared with the human genome and proteome, in order to prevent side effects.

The region of at least 4 consecutive amino acids is identified the same way as described above, however now based on the region being not conserved between the host and the pathogen, *in casu* human and SARS-CoV-2, respectively. The very same methodology can be used, but the identification of the region is made based on difference in sequence instead of homology to be used in the vaccine is preferably not found in the top level pathway classes of the host, more preferably not found in any of the classes. This way, a vaccine can be developed wherein the region of at least 4 consecutive amino acids can be incorporated in a synthetic peptide providing for an epitope not shared by the host.

In an attractive embodiment, the said vaccine also comprises one or more synthetic peptides identified as possible medicament for treatment of a pathway-associated disease as described above. Such a combination provides for an effective epitope as antigen for the host to elicit an immune response, as well as one or more medicaments effective against any side effect brought about by the vaccine. Such a medicament is preferably intended to be administered to a patient in need thereof in a single composition, or as separate compositions, simultaneously, or subsequently.

Again, according to the same methodology, a vaccine against virtually any infectious pathogen can be developed. In step a., the proteome of the host and that of the pathogen are compared in order to identify regions of at least 4 consecutive amino acids, that are not shared, and in step b., one or more pathway classes are identified from a systematic database as described above, comprising pathway classes and protein data of the host.

Proteome data can e.g. be obtained by sequencing the genome of the pathogen and deduct the proteins encoded by the said genome. In many cases it is possible to obtain such proteome data from publicly available databases e.g. SWISSPROT, Uniprot (https://www.uniprot.org/), NCBI (https://www.ncbi.nlm.nih.gov/protein/), EMBL-EBI (https://www.ebi.ac.uk/), SIB (https://www.expasy.org/) and HUGO (https://www.genenames.org/). The skilled person will be aware of other databases that can be used for the envisaged aim.

For the proteome comparison between the infectious pathogen and the host, *in casu* SARS-CoV-2 and human, respectively, any suitable tool known to the skilled person can be used. The proteome comparison in stap a. is preferably performed by perfect matching, in particular using SIB ExPASy tools.

The systematic database comprising a plurality of physiological pathway classes as described above are preferably provided by one or more appropriate databases, in particular STRING (https://string-db.org/), Reactome (https://reactome.org), KEGG (https://www.genome.jp/kegg/), IntAct (https://www.ebi.ac.uk/intact/) and Ingenuity (https://digitalinsights.qiagen.com/). However, any other collection of databases dealing with genomes, biological pathways, diseases, drugs, chemical substances, or genomic and proteomic interactions for bioinformatics research and education, including data analysis in genomics, metagenomics, metabolomics, interactomics, and other omics studies, modelling and simulation in systems biology, and translational research in drug development, known to the skilled person can be used.

The identification of one or more physiological pathways in step b. is preferably done based on a false discovery rate of 0.05 or less, preferably of 10⁻⁴ or less, more preferably of 10⁻⁵ or less, preferably of 10⁻⁶ or less.

Preferably, in step b., the pathway identification is performed by a 100% identity comparison of the conserved consecutive amino acids with the systematic database used.

In an attractive embodiment, the region of at least 4 consecutive amino acids is present in one or more proteins that belong to at least 5 different pathway classes. Such a medicament will be effective in a broad range of diseases and can effectively be used against symptoms of COVID-19.

The one or more amino acid regions preferably comprise at least 5 consecutive amino acids, or 6 consecutive amino acids, or even 7 or 8 consecutive amino acids. As explained herein, the number of identified regions decrease with increasing length of the region, but b the other hand, the longer the region, the more specific and narrower the number of identified pathways will be. A limited pathway number may be desired when a specific medicament is sought to treat a dysfunction in a pathway, where the corresponding amino acid region is also present in other pathways.

Peptide sequences as identified herein, relating to either a specific human pathway or group of human pathways can interfere with the host protein-protein interactions by known mechanisms, for example by orthosteric competition with the associated epitope(s) and/or binding or competition with allosteric sites. Such peptide sequences can be synthesized according to known procedures and used for the treatment of COVID-19 patients, and patients with other diseases with pathologies and/or complications shared with COVID-19 patients. These pharmaceuticals will also be useful for the treatment of SARS-CoV-2 infection and related pathologies.

The conserved peptide sequences identified herein can be used for the functional modulation of these pathways. Hence, the peptides claimed in this patent are not just useful for the treatment of COVID-19, but several of these are also applicable for the treatment of separate viral and non-viral diseases that share mechanisms, pathology or complications with COVID-19 e.g. (a)septic shock, organ failure, particularly for renal- and heart failure, ischemia-reperfusion injury, addiction, psychiatric disease, Alzheimer disease and related dementias, and for antivirals treatment.

Moreover, by the identified shared peptide sequences, biological processes and their pathways that play a crucial role in the clinical manifestations and complications of COVID-19 are identified. This is valuable in drug repurposing as several of the identified biological processes and their pathways can also be targeted by known drugs. This approach can also be used for finding appropriate peptides for the treatment of various manifestations of cancer, based on the analysis of the conserved parts of the genome and proteome of oncogenic viruses (e.g. HPV, HIV), and comparison with the human genome and proteome.

In the above four independent in silico approaches a number of peptide pharmaceuticals were identified. These in silico approaches are (1) selection based on shared SARS-CoV-2 sequences and the human complete proteome (COVID-19); (2) selection based on shared SARS-CoV-2 sequences and human proteins involved in aging-biological process (longevity/aging); (3) selection based on shared SARS-CoV-2 sequences and human proteins involved in 'viral life cycle'-biological process (viral life cycle); (4) selection based on shared SARS-CoV-2 sequences and human proteins involved in 'viral integration'-pathways (viral integration).

The identified peptides from the above approaches, namely based on COVID-19 and the physiological classes of longevity/aging, viral life cycle and viral integration are listed in Table 5. Some SARS-CoV-2 shared peptide sequences in human pathway classes linked to above all four groups, such as AVGLE, ALLLL, LLLVA and LVLLP, and other peptide sequences in fewer classes, such as GAGAA being present in COVID-19, longevity/aging and viral life cycle class, or HLLLVA being present in COVID-19 and viral integration class, or even in a single class. E.g. GVLPQL is present in COVID-19 class only, FIKRS is present in longevity/aging class only, GDAAL is present in viral life cycle class only, and IIFWFS is present in the viral integration class only, see also Tables 5 and 10.

Table 5 shows the values of each of the pharmaceutical peptide the extend that the peptide efficacy to interfere with pathways involved in each of the four groups. For example, the peptide sequence AVGLE and LLLVA show high values in all four groups implying that this peptide interferes with many pathways belonging to each of the four groups. The peptides ALLLL and LVLLP, while interfering with the pathways of all four groups, are less efficient in interfering with the pathways of COVID-19. The differential values for each of the peptides in Table 5 show their differential preference for application in COVID-19 and other viral infections. The value in the column Longevity/aging is to be taken in account dependent on the severity of the disease, e.g. the higher the value in this column the more preferred the use of the peptide in severe cases of the disease.

Table 10 gives a summary of identified peptides for the treatment of COVID-19 patients (class COVID-19), aging related diseases (class Longevity/aging) and patients with viral diseases in general (classes Viral life cycle and Viral integration). This Table lists the peptides to be used as pharmaceuticals for the treatment of COVID-19 and/or Longevity/aging and/or Viral life cycle and/or Viral integration or COVID-19 only or Longevity/aging only or Viral life cycle only or Viral integration only.

The preferred peptide for use as pharmaceutical for the treatment of severely ill COVID-19 patients or patient with any other viral disease are the following peptides: GVLPQL, GVLPQ, LQLPQ, VLQLPQ, AALGVL, VAVGLE, VLQLPQG, ALGVL, AALGV, AVGLE, LLLVA, VAVGL, LLVAA, QLPQG, VLQLP, VLPQL, LLLVAA.

**Table 10: Peptides for use in COVID-19 and related diseases**

| **Class** | **Pharmaceutical peptides (SEQ)** |
|---|---|
| COVID-19, Longevity/aging, Viral life cycle, Viral integration | AVGLE, ALLLL, LLLVA, LVLLP |
| COVID-19, Longevity/aging, Viral life cycle | VAVGL, GAGAA, LGVLV, LLQAP, LLVAA, VLLPL |
| COVID-19, Longevity/aging, Viral integration | VLGLA, GLHLL, SSSRS, LALGG, ALGVL, LPVLQV, QLPQG |
| COVID-19, Viral life cycle, Viral integration | LLPLV, VLQAV, LVLLPL, LGGLH |
| COVID-19, Longevity/aging | VLDLL, EEEQE, AALGV, VLQLP, LDLLV |
| COVID-19, Viral life cycle | SSCSS, LLVAAG, LQAVG, VLPQL, LVAAG |
| COVID-19, Viral integration | VLLPLV, HLLLVA, SSRSR, SCSSS, SSSRSR, HLLLVAA, LALLLL, VLGLAA, SSRSSS, LGGLHL, AALGVL, LALLL, RLALGG, VAVGLE, LLLVAA, SSCSSS, VLQLPQG, GGLHLL, CSSSRS, SCSSSR, SSCSSSR, VLQAVG |
| Longevity/aging, Viral life cycle | VADGL, LVAVG, DGVDV, VLQPE, AVLLA, AADPA, GLAAV, LVLLL, VGVAL, LVGVL, VVEVV, VLLLL, LLLQL, EEAAR, VAAVL, SSLSS, LLLLV |
| COVID-19 | GVLPQL, LGVLVP, VLDLLV, LPLQL, AADLDD, LQLPQ, GGLHL, PAADL, ADLDD, PAADLD, AGAAL, GAGAAL, AADLD, GVLPQ, VLQLPQ, LPLQLG |
| Longevity/aging | FIKRS, RQVLL, LDRLE, KIKTI, VLVPL, PVPEV, LGLLP |
| Viral life cycle | GDAAL, LTSFS, LHVVG, TFSSIF, FSSIF, QPGVA, EQEED, VDGQV, TFSSI, PLVEQ |
| Viral integration | IIFWFS, IIFWF |

The preferred pharmaceutical peptide sequences that are to be used in case of diseases related to Viral integration are: IIFWFS, IIFWF, LLLVAA, AALGVL, LALLL, LALLLL, VLLPLV, ALLLL.

The preferred pharmaceutical peptide sequences that are to be used in case of diseases related to Viral life cycle are: TFSSIF, FSSIF, TFSSI.

The preferred pharmaceutical peptide sequences that are to be used in case of diseases related to Longevity/aging are: VLVPL, FIKRS, PVPEV, KIKTI, VADGL, LLLLV, ALGVL, AADPA, GLAAV.

The preferred pharmaceutical peptide sequences that are to be used in the prevention and treatment of early phase of COVID-19 or any other viral infection are: AALGVL, LLLVAA, VAVGLE, VLQLPQG, LLLVA, AVGLE, VLPQL.

The preferred pharmaceutical peptide sequences that are to be used in the prevention and treatment of COVID-19 or any other viral infection are: AVGLE, LLLVA, LLPLV, LGGLH.

The preferred pharmaceutical peptide sequences that are to be used in the prevention and treatment of geriatric diseases and frailty are: VADGL, DGVDV, AVLLA, GLAAV, LLLLV, AALGV, ALGVL.

In order to select peptides from Table 5 that can be used for the treatment of COVID-19 associated pathologies as mentioned in Figure 1, proteins can be identified from any protein(gene)-disease associated database that is linked to the pathologies in Figure 1. Such database contains curated and inferred gene-disease associations. Curated protein(gene)-disease associations are extracted from the published literature. Inferred associations are established via curated chemical-gene interactions (e.g., gene A is associated with disease B because gene A has a curated interaction with chemical C, and chemical C has a curated association with disease B).

This way, peptides that are shared in the human diseases/complications (as shown in Figure 1) - associated proteins linked to COVID-19 associated pathologies.

Table 11 shows list of SARS-CoV-2 shared peptide sequences found in human protein-disease database that are involved in the pathologies mentioned in Figure 1, independent of whether these pathologies occur in the context of COVID-19. These shared peptides can be used as pharmaceuticals for the treatment of the in Figure 1 mentioned COVID-19 associated pathologies.

**Table 11: SARS-CoV-2 shared peptide sequences found in human protein-disease database related to ageing and longevity**

| | | | | |
|---|---|---|---|---|
| ALGVL | ALLLL | AVGLE | VAVGL | EEAAR |
| EQEED | FIKRS | GLAAV | IIFWF | LGVLV |
| LLLQL | LLLVA | LLPLV | PAADL | PVPEV |
| QLPQG | VLQLP | SRSSS | SSCSS | VDGQV |

Peptides that are shared between the database for human disease-associated proteins and the COVID-19-associated pathologies shown in Figure 1 were selected. Table 11 shows the list of shared peptides that emerged from this comparison. All the 20 peptides in this list can be successfully used as pharmaceuticals for the treatment of the in Figure 1 mentioned pathologies. However, not all 20 peptides are equally effective for use in the treatment of the pathologies of the Groups 1a, 1b, 2 and 3.

For the pathologies of the Groups 1a and 1b especially the peptides ALGVL, LGVLV, VLQLP, LLPLV, QLPQG, AVGLE, VAVGL, and LLLQL are effective. For use for the treatment of Group 2, inflammation, the peptides SRSSS, VAVGL, LLLQL, ALGVL, LGVLV, EEAAR, VLQLP, QLPQG and ALLLL are effective, while for use in the treatment of the pathologies of Group 3 especially the peptides ALGVL, EEAAR, IIFWF, SSCSS, VAVGL and VLQLP are effective.

In a following aspect, the invention relates to a method for the preparation of a medicament against a pathogen infection or against a pathology related to the said infection, the medicament comprising a synthetic peptide comprising a region comprising at least 4 consecutive amino acids, comprising the steps of:
a. identification of one or more conserved regions of at least 4 consecutive amino acids with a 100% match between the proteome of the pathogen and the proteome of the host;
b. identification of at least one pathway class from a systematic database comprising a plurality of physiological pathway classes of the said host, each class comprising a plurality of host proteins that are functionally related to the said physiological pathway, wherein the said at least one pathway class shares at least one pathology and/or complication of the infection as a result of dysfunction in the said pathway and comprises at least one host protein comprising regions of at least 4 consecutive amino acids that are conserved with a match of 100% between the pathogen proteome and the host proteome; and
c. selecting the said identified conserved region of at least 4 consecutive amino acids of step a), present in the at least one protein comprised in the pathway class identified in step b) for the preparation of the medicament.

Such a method is based on the principle described herein and provides a very elegant way to determine suitable amino acid regions that can be used in synthetic peptides for the preparation of a medicament, without being bound to a single pathogen or host.

As discussed above, step a. can be replaced by steps a1 and a2,

| | |
|---|---|
| step a1. | comprising identification of one or more biological processes from a systematic database, the database comprising a plurality of biological |
| | processes of the host and, for each biological process, a plurality of host proteins that are functionally related to the said biological process, and |
| step a2. | comprising identification of at least one conserved region of at least 4 consecutive amino acids in one or more of the plurality of proteins identified in step a1., said conserved region of at least 4 consecutive amino acids having a 100% match with the pathogen proteome. |

In a following embodiment, the invention relates to a method for the preparation of a host vaccine against a pathogen infection, the vaccine comprising a synthetic peptide comprising a region comprising at least 4 consecutive amino acids, comprising the steps of:
a. identification of at least one region of at least 4 consecutive amino acids not conserved between the pathogen proteome and the host proteome; the said at least one region of at least 4 consecutive amino acids not being present in one or more proteins belonging to at least one pathway class from a systematic database comprising a plurality of physiological pathway classes of the host, each class comprising a plurality of host proteins that are functionally related to the said physiological pathway;
b. selecting the said region of at least 4 consecutive amino acids for the preparation of the vaccine.

As indicated above, the method as described herein can conveniently be used for the identification of suitable epitopes and the preparation of synthetic peptides comprising such epitopes, without being bound to a single pathogen or host. The vaccine can also comprise one or more medicaments as identified with the above method in order to reduce side effects.

### Examples

*In vitro* as well as *in vivo* experiments support the treatment efficacy of the selected peptide pharmaceuticals in various important aspects of COVID-19 and its related pathologies.

### Inhibition of SARS-CoV-2 replication in human pulmonary epithelial cells by peptides AVGLE, LLLVAA, and LLPLV.

Human conducting airway epithelial cells (Calu-3 cells or other appropriate airway epithelial cells) were cultured on Transwell membranes for more than 10 days (according to Tseng et al., J. Virol. 79:9470-9, 2005) in order to generate cultures of highly polarized cells connected by tight junctions and microvilli. Cultures were then inoculated with SARS-CoV-2 via the apical surface. Four groups of 40 cultures were set up. One (control) group was not supplemented with peptide, the second group was supplemented with peptide AVGLE (10 mcg/ml), the third group was supplemented with peptide LLLVAA and the fourth group was supplemented with peptide LLPLV.

At 10, 24, 34, and 48 hrs after inoculation the apical washes were harvested from 10 cultures of each group, and the live virus titres were determined on VeroE6 cells. The peptides AVGLE, LLLVAA (10 mcg/ml) and LLPLV (10 mcg/ml) reduced the live virus titres at 24, 34 and 48 hrs after inoculation of the cultures, while the control group did not.

### Inhibition of SARS-CoV-2 infection of human PBMC

Infection of short term human PBMC cultures with SARS-CoV-2 supplemented with various peptides (10 mcg/ml) and cellular signalling pathways were analysed by gene array. Control cultures a\were not supplemented with peptide. The following peptides were evaluated: AVGLE, LLLVA, VLPQL, ALGVL, TFSSIF, TFSSI, FSSIF.

The results show that these peptide specially affect genes that are influenced by the SARS-CoV-2, resulting in the change in the activity of various pathways, such as insulin signalling pathway, glutamatergic synapse, platelet activation and apelin signalling pathway.

### Inhibition of LPS and TSST-1 induced COVID-19 related pathology by various peptides

LPS (endotoxin) and TSST-1 (exotoxin) induced shock in mice are widely accepted models for human sepsis and related pathology (Group 1a and Group 1b in Figure 1). Similar pathology is frequently seen in severe cases of COVID-19. In the LPS model the systemic effects are largely mediated by macrophages, while in the TSST-1 model the systemic effects are mediated by T cells and antigen-presenting cells. In both models the systemic shock is due to an excessive inflammatory cytokine release (so-called cytokine storm) accompanied by endogenous mediators, including oxidative stress mediators, leading to characteristic pathophysiological reactions such as fever, leukopenia, thrombocytopenia, hemodynamic changes and disseminated intravascular coagulation as well as leukocyte infiltration and inflammation of various organs, all of which can ultimately lead to death (Khan et al., Clin Exp Immunol 160, 466-78, 2010).

In the LPS model, 8-10 weeks old BALB/c mice were injected intraperitoneally (i.p.) with 10 mg/kg LPS in saline. Control mice received saline i.p. only. In the TSST-1 model, 8-10 weeks old BALB/c mice were injected with 25 mg/kg D-Galactosamine in saline i.p. On the same day the latter mice are injected with 8 mcg TSST-1 subcutaneously (s.c.). Control groups are injected with either TSST-1 s.c. or D-Galactosamine i.p. only.

In both models, mice (n=8) were pre-treated with peptide (10 mg/kg) 2 hrs before injection of either LPS or D-Galactosamine/TSST-1 or treated 12 hrs after injection of either of these shock-inducing agents. In both models, sickness development is semi-quantitatively measured as described earlier (Khan et al., Hum Immunol 62, 1-7, 2002).

All peptides of Table 12 reduced septic shock and mortality in both models as compared to mice not treated with peptide, but the peptides ALGVL, ALLLL, LLPLV, EEAAR, LLLQL, VAVGL, VLQLP reduced the clinical features of the septic shock more than the other peptides. Determination of the inflammatory mediators TNF-a and IL-6 showed that the latter peptides were more effective in reducing the release of these mediators than the other peptides.

### Amelioration of ischemia-reperfusion injury by peptides

The peptides ALGVL, EEAAR, IIFWF, GLAAV, SSCSS, VAVGL, QLPQG, VLQLP and AVGLE inhibit the ischemia-reperfusion injury in 12-16 weeks old C57BL mice. In this model, the mice were anaesthetized by isoflurane inhalation, and the left renal pedicle clamped for 25 minutes using an atraumatic micro-vascular clamp as described before (Khan et al., Nephrol Dial Transplant 24, 2701-8, 2009). After release of the clamp, restoration of blood flow was inspected visually, and a contra-lateral nephrectomy was performed. The abdominal wound was closed, and the mice were given rest to recover for 3 days. After 3 days, survival of control mice (n=10) not treated with a peptide is 50%, while mice (n=10 per peptide) treated with either ALGVL, EEAAR, IIFWF, GLAAV, SSCSS, VAVGL, QLPQG, VLQLP and AVGLE (10 mg/kg) survive 100%. This model is generally accepted as a representative model for the evaluation of the effectiveness of candidate pharmaceuticals for use in the treatment of other reperfusion-ischemia pathologies as listed in Group 3 of Figure 1.

## Claims

1. Medicament for treatment of COVID-19 and related pathologies, the said medicament comprising or interacting with one or more conserved regions of at least 4 consecutive amino acids present with a 100% match in both the SARS-CoV-2 proteome and the human proteome.

2. Medicament of claim 1, wherein the one or more conserved regions of at least 4 consecutive amino acids with a 100% match are selected by:
a. identification of one or more conserved regions of at least 4 consecutive amino acids with a 100% match between the SARS-CoV-2 proteome and the human proteome;
b. identification of at least one pathway class from a systematic database comprising a plurality of human physiological pathway classes, each class comprising a plurality of human proteins that are functionally related to the said physiological pathway, wherein the said at least one pathway class shares at least one pathology and/or complication of the COVID-19 infection as a result of dysfunction in the said pathway and comprises at least one human protein comprising one or more conserved regions of at least 4 consecutive amino acids that have a 100% match with the SARS-CoV-2 proteome; and
c. selecting the said identified one or more conserved regions of at least 4 consecutive amino acids of step a., present in the at least one protein comprised in the pathway class identified in step b. for the preparation of the medicament.

3. Medicament of claim 2, wherein step a. is replaced by steps a1. and a2.,
| | |
|---|---|
| step a1. | comprising identification of one or more biological processes from a systematic database, the database comprising a plurality of human biological processes and, for each biological process, a plurality of human proteins that are functionally related to the said biological process, and |
| step a2. | comprising identification of at least one conserved region of at least 4 consecutive amino acids in one or more of the plurality of proteins |
| | identified in step a1., said conserved region of at least 4 consecutive amino acids having a 100% match with the SARS-CoV-2 proteome. |

4. Medicament being a vaccine against COVID-19, the said vaccine comprising one or more agents interacting with at least one region of at least 4 consecutive amino acids present in the SARS-CoV-2 proteome, the said region of at least 4 consecutive amino acids being selected by:
a. identification of at least one region of at least 4 consecutive amino acids not conserved between the SARS-CoV-2 proteome and the human proteome; and
b. selecting the said at least one region of at least 4 consecutive amino acids for the preparation of the vaccine.

5. Medicament claim 4, wherein step a. comprises:
a. identification of at least one region of at least 4 consecutive amino acids not conserved between the SARS-CoV-2 proteome and the human proteome; the said at least one region of at least 4 consecutive amino acids not being present in one or more proteins belonging to at least one pathway class from a systematic database comprising a plurality of physiological pathway classes, each class comprising a plurality of proteins that are functionally related to the said physiological pathway.

6. Medicament of claim 4 or 5, further comprising a medicament of any of claims 1 - 3, wherein the medicament is intended to be administered to a patient in need thereof in a single composition, or as separate compositions, simultaneously, or subsequently.

7. Medicament of any of the preceding claims, wherein:
a. the proteome data for step a. are obtained or derived from a database, chosen from SWISSPROT, Uniprot, NCBI, EMBL-EBI, SIB and HUGO, a/or
b. the proteome comparison in step a. is performed by perfect matching, in particular using SIB ExPASy tools.

8. Medicament of any of the preceding claims 2, 3, 5, or any claim dependent thereon, wherein:
a. the systematic database comprising a plurality of physiological pathway classes is chosen from the group, consisting of: STRING, Interactome, KEGG, IntAct and Ingenuity; and/ or
b. the physiological pathway is identified by a false discovery rate of 0.05 or less, preferably of 10⁻⁴ or less, more preferably of 10⁻⁵ or less, preferably of 10⁻⁶ or less.

9. Medicament of any of the preceding claims 2, 3, 5 or any claim dependent thereon, wherein
a. the pathway identification in step b. is performed by a 100% identity comparison of the conserved consecutive amino acids with the systematic database, and/or
b. the region of at least 4 consecutive amino acids is present in one or more proteins that belong to at least 5 different pathway classes.

10. Medicament of any of the preceding claims wherein the one or more conserved amino acid regions comprise:
a. at least 5 consecutive amino acids; or
b. 4 to 8 consecutive amino acids.

11. Medicament of claim 10, wherein region of at least 5 consecutive amino acids is chosen from the group, consisting of: ALGVL, ALLLL, AVGLE, EEAAR, EQEED, FIKRS, GAGAA, GLAAV, GLHLL, IIFWF, LALGG, LGGLH, LGVLV, LLLQL, LLLVA, LPVLQV, LLPLV, LLQAV, LLVAA, LVLLPL, PAADL, PVPEV, QLPQG, SRSSS, SSCSS, SSSRS, VDGQV, LVLLP, VAVGL, VLGLA, VLLPL, VLQAV, VLQLP, VLDLL, EEEQE, AALGV, LDLLV, LLVAAG, LQAVG, VLPQL, LVAAG, VLLPLV, HLLLVA, SSRSR, SCSSS, SSSRSR, HLLLVAA, LALLLL, VLGLAA, SSRSSS, LGGLHL, AALGVL, LALLL, RLALGG, VAVGLE, LLLVAA, SSCSSS, VLQLPQG, GGLHLL, CSSSRS, SCSSSR, SSCSSSR, VLQAVG, VADGL, LVAVG, DGVDV, VLQPE, AVLLA, AADPA, LVLLL, VGVAL, LVGVL, VVEVV, VLLLL, VAAVL, SSLSS, LLLLV, GVLPQL, LGVLVP, VLDLLV, LPLQL, AADLDD, LQLPQ, GGLHL, ADLDD, PAADLD, AGAAL, GAGAAL, AADLD, GVLPQ, VLQLPQ, LPLQLG, RQVLL, LDRLE, KIKTI, VLVPL, LGLLP, GDAAL, LTSFS, LHVVG, TFSSIF, FSSIF, QPGVA, TFSSI, PLVEQ, and IIFWFS, preferably from ALGVL, ALLLL, AVGLE, EEAAR, IIFWF, FSSIF, GLAAV, LLPLV, LLLVA, LLLVAA, LLLQL, QLPQG, SSCSS, TFSSI, TFSSIF, VAVGL, VLPQL and VLQLP.

12. Medicament of claim 1 or 2 or any claim dependent thereon, wherein the COVID-19 related diseases are chosen from ischemia, reperfusion, hypoxia, shock, hypovolemia, sepsis, septic shock, inflammation, heart failure, infarction, cardiac, liver and kidney failure and injury, severe acute respiratory syndrome, respiratory distress syndrome, acute lung injury, multiple organ failure, metabolic syndrome, acute or chronic heart, kidney, lung and liver disease, acute or chronic inflammatory disease, longevity and aging diseases or symptoms related to or characteristic for these diseases.

13. Method for the preparation of a medicament against a pathogen infection or against a pathology related to the said infection, the medicament comprising a synthetic peptide comprising a region comprising at least 4 consecutive amino acids, comprising the steps of:
a. identification of one or more conserved regions of at least 4 consecutive amino acids with a 100% match between the proteome of the pathogen and the proteome of the host;
b. identification of at least one pathway class from a systematic database comprising a plurality of physiological pathway classes of the said host, each class comprising a plurality of host proteins that are functionally related to the said physiological pathway, wherein the said at least one pathway class shares at least one pathology and/or complication of the infection as a result of dysfunction in the said pathway and comprises at least one host protein comprising regions of at least 4 consecutive amino acids that are conserved with a match of 100% between the pathogen proteome and the host proteome; and
c. selecting the said identified conserved region of at least 4 consecutive amino acids of step a), present in the at least one protein comprised in the pathway class identified in step b) for the preparation of the medicament.

14. Method of claim 13, wherein step a. is replaced by steps a1 and a2,
| | |
|---|---|
| step a1. | comprising identification of one or more biological processes from a systematic database, the database comprising a plurality of biological processes of the host and, for each biological process, a plurality of host proteins that are functionally related to the said biological process, and |
| step a2. | comprising identification of at least one conserved region of at least 4 consecutive amino acids in one or more of the plurality of proteins identified in step a1., said conserved region of at least 4 consecutive amino acids having a 100% match with the pathogen proteome. |

15. Method for the preparation of a host vaccine against a pathogen infection, the vaccine comprising a peptide comprising a region comprising at least 4 consecutive amino acids, comprising the steps of:
a. identification of at least one region of at least 4 consecutive amino acids not conserved between the pathogen proteome and the host proteome; the said at least one region of at least 4 consecutive amino acids not being present in one or more proteins belonging to at least one pathway class from a systematic database comprising a plurality of physiological pathway classes of the host, each class comprising a plurality of host proteins that are functionally related to the said physiological pathway;
b. selecting the said region of at least 4 consecutive amino acids for the preparation of the vaccine.


SEQUENCE LISTING

<110>  Unthreadt BV

<120>  Medicament for treatment of COVID-19

<130>  Unthreadt1

<160>  135

<170>  PatentIn version 3.5

<210>  1
<211>  5
<212>  PRT
<213>  Homo sapiens

<400>  1

Ala Ala Asp Leu Asp
1               5

<210>  2
<211>  6
<212>  PRT
<213>  Homo sapiens

<400>  2

Ala Ala Asp Leu Asp Asp
1               5

<210>  3
<211>  5
<212>  PRT
<213>  Homo sapiens

<400>  3

Ala Ala Asp Pro Ala
1               5

<210>  4
<211>  5
<212>  PRT
<213>  Homo sapiens

<400>  4

Ala Ala Leu Gly Val
1               5

<210>  5
<211>  6
<212>  PRT
<213>  Homo sapiens

<400>  5

Ala Ala Leu Gly Val Leu
1               5

<210>  6
<211>  5
<212>  PRT
<213>  Homo sapiens

<400>  6

Ala Asp Leu Asp Asp
1               5

<210>  7
<211>  5
<212>  PRT
<213>  Homo sapiens

<400>  7

Ala Gly Ala Ala Leu
1               5

<210>  8
<211>  5
<212>  PRT
<213>  Homo sapiens

<400>  8

Ala Leu Gly Val Leu
1               5

<210>  9
<211>  5
<212>  PRT
<213>  Homo sapiens

<400>  9

Ala Leu Leu Leu Leu
1               5

<210>  10
<211>  5
<212>  PRT
<213>  Homo sapiens

<400>  10

Ala Val Gly Leu Glu
1               5

<210>  11
<211>  5
<212>  PRT
<213>  Homo sapiens

<400>  11

Ala Val Leu Leu Ala
1               5


<210>  12
<211>  6
<212>  PRT
<213>  Homo sapiens

<400>  12

Cys Ser Ser Ser Arg Ser
1               5


<210>  13
<211>  5
<212>  PRT
<213>  Homo sapiens

<400>  13

Asp Gly Val Asp Val
1               5


<210>  14
<211>  5
<212>  PRT
<213>  Homo sapiens

<400>  14

Glu Glu Ala Ala Arg
1               5


<210>  15
<211>  5
<212>  PRT
<213>  Homo sapiens

<400>  15

Glu Glu Glu Gln Glu
1               5


<210>  16
<211>  5
<212>  PRT
<213>  Homo sapiens

<400>  16

Glu Gln Glu Glu Asp
1               5


<210>  17

<211>  5
<212>  PRT
<213>  Homo sapiens

<400>  17

Phe Ile Lys Arg Ser
1               5


<210>  18
<211>  5
<212>  PRT
<213>  Homo sapiens

<400>  18

Phe Ser Ser Ile Phe
1               5


<210>  19
<211>  5
<212>  PRT
<213>  Homo sapiens

<400>  19

Gly Ala Gly Ala Ala
1               5


<210>  20
<211>  6
<212>  PRT
<213>  Homo sapiens

<400>  20

Gly Ala Gly Ala Ala Leu
1               5


<210>  21
<211>  5
<212>  PRT
<213>  Homo sapiens

<400>  21

Gly Asp Ala Ala Leu
1               5


<210>  22
<211>  5
<212>  PRT
<213>  Homo sapiens

<400>  22

Gly Gly Leu His Leu
1               5

<210>  23
<211>  6
<212>  PRT
<213>  Homo sapiens

<400>  23

Gly Gly Leu His Leu Leu
1               5


<210>  24
<211>  5
<212>  PRT
<213>  Homo sapiens

<400>  24

Gly Leu Ala Ala Val
1               5


<210>  25
<211>  5
<212>  PRT
<213>  Homo sapiens

<400>  25

Gly Leu His Leu Leu
1               5


<210>  26
<211>  5
<212>  PRT
<213>  Homo sapiens

<400>  26

Gly Val Leu Pro Gln
1               5


<210>  27
<211>  6
<212>  PRT
<213>  Homo sapiens

<400>  27

Gly Val Leu Pro Gln Leu
1               5


<210>  28
<211>  6
<212>  PRT
<213>  Homo sapiens

<400>  28

His Leu Leu Leu Val Ala
1               5


<210>  29
<211>  7
<212>  PRT
<213>  Homo sapiens

<400>  29

His Leu Leu Leu Val Ala Ala
1               5


<210>  30
<211>  5
<212>  PRT
<213>  Homo sapiens

<400>  30

Ile Ile Phe Trp Phe
1               5


<210>  31
<211>  6
<212>  PRT
<213>  Homo sapiens

<400>  31

Ile Ile Phe Trp Phe Ser
1               5


<210>  32
<211>  5
<212>  PRT
<213>  Homo sapiens

<400>  32

Lys Ile Lys Thr Ile
1               5


<210>  33
<211>  5
<212>  PRT
<213>  Homo sapiens

<400>  33

Leu Ala Leu Gly Gly
1               5


<210>  34
<211>  5
<212>  PRT

<213>  Homo sapiens

<400>  34

Leu Ala Leu Leu Leu
1               5

<210>  35
<211>  6
<212>  PRT
<213>  Homo sapiens

<400>  35

Leu Ala Leu Leu Leu Leu
1               5

<210>  36
<211>  5
<212>  PRT
<213>  Homo sapiens

<400>  36

Leu Asp Leu Leu Val
1               5

<210>  37
<211>  5
<212>  PRT
<213>  Homo sapiens

<400>  37

Leu Asp Arg Leu Glu
1               5

<210>  38
<211>  5
<212>  PRT
<213>  Homo sapiens

<400>  38

Leu Gly Gly Leu His
1               5

<210>  39
<211>  6
<212>  PRT
<213>  Homo sapiens

<400>  39

Leu Gly Gly Leu His Leu
1               5

<210>  40
<211>  5
<212>  PRT
<213>  Homo sapiens

<400>  40

Leu Gly Leu Leu Pro
1               5


<210>  41
<211>  5
<212>  PRT
<213>  Homo sapiens

<400>  41

Leu Gly Val Leu Val
1               5


<210>  42
<211>  6
<212>  PRT
<213>  Homo sapiens

<400>  42

Leu Gly Val Leu Val Pro
1               5


<210>  43
<211>  5
<212>  PRT
<213>  Homo sapiens

<400>  43

Leu His Val Val Gly
1               5


<210>  44
<211>  5
<212>  PRT
<213>  Homo sapiens

<400>  44

Leu Leu Leu Leu Val
1               5


<210>  45
<211>  5
<212>  PRT
<213>  Homo sapiens

<400>  45

Leu Leu Leu Gln Leu

1                   5


<210>  46
<211>  5
<212>  PRT
<213>  Homo sapiens

<400>  46

Leu Leu Leu Val Ala
1                   5


<210>  47
<211>  6
<212>  PRT
<213>  Homo sapiens

<400>  47

Leu Leu Leu Val Ala Ala
1                   5


<210>  48
<211>  5
<212>  PRT
<213>  Homo sapiens

<400>  48

Leu Leu Pro Leu Val
1                   5


<210>  49
<211>  5
<212>  PRT
<213>  Homo sapiens

<400>  49

Leu Leu Gln Ala Pro
1                   5


<210>  50
<211>  5
<212>  PRT
<213>  Homo sapiens

<400>  50

Leu Leu Val Ala Ala
1                   5


<210>  51
<211>  6
<212>  PRT
<213>  Homo sapiens

<400>  51

Leu Leu Val Ala Ala Gly
1               5

<210>  52
<211>  5
<212>  PRT
<213>  Homo sapiens

<400>  52

Leu Pro Leu Gln Leu
1               5

<210>  53
<211>  6
<212>  PRT
<213>  Homo sapiens

<400>  53

Leu Pro Leu Gln Leu Gly
1               5

<210>  54
<211>  6
<212>  PRT
<213>  Homo sapiens

<400>  54

Leu Pro Val Leu Gln Val
1               5

<210>  55
<211>  5
<212>  PRT
<213>  Homo sapiens

<400>  55

Leu Gln Ala Val Gly
1               5

<210>  56
<211>  5
<212>  PRT
<213>  Homo sapiens

<400>  56

Leu Gln Leu Pro Gln
1               5

<210>  57
<211>  5

<212>  PRT
<213>  Homo sapiens

<400>  57

Leu Thr Ser Phe Ser
1               5


<210>  58
<211>  5
<212>  PRT
<213>  Homo sapiens

<400>  58

Leu Val Ala Ala Gly
1               5


<210>  59
<211>  5
<212>  PRT
<213>  Homo sapiens

<400>  59

Leu Val Ala Val Gly
1               5


<210>  60
<211>  5
<212>  PRT
<213>  Homo sapiens

<400>  60

Leu Val Gly Val Leu
1               5


<210>  61
<211>  5
<212>  PRT
<213>  Homo sapiens

<400>  61

Leu Val Leu Leu Leu
1               5


<210>  62
<211>  5
<212>  PRT
<213>  Homo sapiens

<400>  62

Leu Val Leu Leu Pro
1               5

<210>  63
<211>  6
<212>  PRT
<213>  Homo sapiens

<400>  63

Leu Val Leu Leu Pro Leu
1               5


<210>  64
<211>  5
<212>  PRT
<213>  Homo sapiens

<400>  64

Pro Ala Ala Asp Leu
1               5


<210>  65
<211>  6
<212>  PRT
<213>  Homo sapiens

<400>  65

Pro Ala Ala Asp Leu Asp
1               5


<210>  66
<211>  5
<212>  PRT
<213>  Homo sapiens

<400>  66

Pro Leu Val Glu Gln
1               5


<210>  67
<211>  5
<212>  PRT
<213>  Homo sapiens

<400>  67

Pro Val Pro Glu Val
1               5


<210>  68
<211>  5
<212>  PRT
<213>  Homo sapiens

<400>  68

Gln Leu Pro Gln Gly
1               5

<210>  69
<211>  5
<212>  PRT
<213>  Homo sapiens

<400>  69

Gln Pro Gly Val Ala
1               5

<210>  70
<211>  6
<212>  PRT
<213>  Homo sapiens

<400>  70

Arg Leu Ala Leu Gly Gly
1               5

<210>  71
<211>  5
<212>  PRT
<213>  Homo sapiens

<400>  71

Arg Gln Val Leu Leu
1               5

<210>  72
<211>  5
<212>  PRT
<213>  Homo sapiens

<400>  72

Ser Cys Ser Ser Ser
1               5

<210>  73
<211>  6
<212>  PRT
<213>  Homo sapiens

<400>  73

Ser Cys Ser Ser Ser Arg
1               5

<210>  74
<211>  5
<212>  PRT
<213>  Homo sapiens

<400>  74

Ser Ser Cys Ser Ser
1               5

<210>  75
<211>  6
<212>  PRT
<213>  Homo sapiens

<400>  75

Ser Ser Cys Ser Ser Ser
1               5

<210>  76
<211>  7
<212>  PRT
<213>  Homo sapiens

<400>  76

Ser Ser Cys Ser Ser Ser Arg
1               5

<210>  77
<211>  5
<212>  PRT
<213>  Homo sapiens

<400>  77

Ser Ser Leu Ser Ser
1               5

<210>  78
<211>  5
<212>  PRT
<213>  Homo sapiens

<400>  78

Ser Ser Arg Ser Arg
1               5

<210>  79
<211>  6
<212>  PRT
<213>  Homo sapiens

<400>  79

Ser Ser Arg Ser Ser Ser
1               5

<210>  80

<211>  5
<212>  PRT
<213>  Homo sapiens

<400>  80

Ser Ser Ser Arg Ser
1               5


<210>  81
<211>  6
<212>  PRT
<213>  Homo sapiens

<400>  81

Ser Ser Ser Arg Ser Arg
1               5


<210>  82
<211>  5
<212>  PRT
<213>  Homo sapiens

<400>  82

Thr Phe Ser Ser Ile
1               5


<210>  83
<211>  6
<212>  PRT
<213>  Homo sapiens

<400>  83

Thr Phe Ser Ser Ile Phe
1               5


<210>  84
<211>  5
<212>  PRT
<213>  Homo sapiens

<400>  84

Val Ala Ala Val Leu
1               5


<210>  85
<211>  5
<212>  PRT
<213>  Homo sapiens

<400>  85

Val Ala Asp Gly Leu
1               5

<210>  86
<211>  5
<212>  PRT
<213>  Homo sapiens

<400>  86

Val Ala Val Gly Leu
1               5


<210>  87
<211>  6
<212>  PRT
<213>  Homo sapiens

<400>  87

Val Ala Val Gly Leu Glu
1               5


<210>  88
<211>  5
<212>  PRT
<213>  Homo sapiens

<400>  88

Val Asp Gly Gln Val
1               5


<210>  89
<211>  5
<212>  PRT
<213>  Homo sapiens

<400>  89

Val Gly Val Ala Leu
1               5


<210>  90
<211>  5
<212>  PRT
<213>  Homo sapiens

<400>  90

Val Leu Asp Leu Leu
1               5


<210>  91
<211>  6
<212>  PRT
<213>  Homo sapiens

<400>  91

Val Leu Asp Leu Leu Val
1               5

<210>  92
<211>  5
<212>  PRT
<213>  Homo sapiens

<400>  92

Val Leu Gly Leu Ala
1               5

<210>  93
<211>  6
<212>  PRT
<213>  Homo sapiens

<400>  93

Val Leu Gly Leu Ala Ala
1               5

<210>  94
<211>  5
<212>  PRT
<213>  Homo sapiens

<400>  94

Val Leu Leu Leu Leu
1               5

<210>  95
<211>  5
<212>  PRT
<213>  Homo sapiens

<400>  95

Val Leu Leu Pro Leu
1               5

<210>  96
<211>  6
<212>  PRT
<213>  Homo sapiens

<400>  96

Val Leu Leu Pro Leu Val
1               5

<210>  97
<211>  5
<212>  PRT

<213>  Homo sapiens

<400>  97

Val Leu Pro Gln Leu
1               5


<210>  98
<211>  5
<212>  PRT
<213>  Homo sapiens

<400>  98

Val Leu Gln Ala Val
1               5


<210>  99
<211>  6
<212>  PRT
<213>  Homo sapiens

<400>  99

Val Leu Gln Ala Val Gly
1               5


<210>  100
<211>  5
<212>  PRT
<213>  Homo sapiens

<400>  100

Val Leu Gln Leu Pro
1               5


<210>  101
<211>  6
<212>  PRT
<213>  Homo sapiens

<400>  101

Val Leu Gln Leu Pro Gln
1               5


<210>  102
<211>  7
<212>  PRT
<213>  Homo sapiens

<400>  102

Val Leu Gln Leu Pro Gln Gly
1               5

<210>  103
<211>  5
<212>  PRT
<213>  Homo sapiens

<400>  103

Val Leu Gln Pro Glu
1               5


<210>  104
<211>  5
<212>  PRT
<213>  Homo sapiens

<400>  104

Val Leu Val Pro Leu
1               5


<210>  105
<211>  5
<212>  PRT
<213>  Homo sapiens

<400>  105

Val Val Glu Val Val
1               5


<210>  106
<211>  9
<212>  PRT
<213>  Unknown

<220>
<223>  SARS-CoV-2

<400>  106

Leu Leu Pro Ala Ala Asp Leu Asp Asp
1               5


<210>  107
<211>  9
<212>  PRT
<213>  Unknown

<220>
<223>  SARS-COV-2

<400>  107

Ser Ser Cys Ser Ser Ser Arg Ser Arg
1               5


<210>  108
<211>  9

<212>  PRT
<213>  Unknown

<220>
<223>  SARS-COV-2

<400>  108

Ser Ser Arg Ser Ser Ser Arg Ser Arg
1               5


<210>  109
<211>  7
<212>  PRT
<213>  Unknown

<220>
<223>  SARS-COV-2

<400>  109

Leu Val Leu Leu Pro Leu Val
1               5


<210>  110
<211>  6
<212>  PRT
<213>  Unknown

<220>
<223>  SARS-COV-2

<400>  110

Gly Ala Gly Ala Ala Leu
1               5


<210>  111
<211>  6
<212>  PRT
<213>  Unknown

<220>
<223>  SARS-COV-2

<400>  111

Val Leu Pro Pro Leu Leu
1               5


<210>  112
<211>  6
<212>  PRT
<213>  Unknown

<220>
<223>  SARS-COV-2

<400>  112

Gly Gly Val Ala Gly Ala
1               5

<210>  113
<211>  6
<212>  PRT
<213>  Unknown

<220>
<223>  SARS-COV-2

<400>  113

Ala Ala Leu Gly Val Leu
1               5

<210>  114
<211>  6
<212>  PRT
<213>  Unknown

<220>
<223>  SARS-COV-2

<400>  114

Asp Pro Ala Gln Leu Pro
1               5

<210>  115
<211>  6
<212>  PRT
<213>  Unknown

<220>
<223>  SARS-COV-2

<400>  115

Leu Leu Gly Val Gly Gly
1               5

<210>  116
<211>  6
<212>  PRT
<213>  Unknown

<220>
<223>  SARS-COV-2

<400>  116

Leu Gly Val Leu Val Pro
1               5

<210>  117
<211>  7

<212>  PRT
<213>  Unknown

<220>
<223>  SARS-COV-2

<400>  117

Val Leu Gln Ala Val Gly Ala
1               5


<210>  118
<211>  8
<212>  PRT
<213>  Unknown

<220>
<223>  SARS-COV-2

<400>  118

Gly Val Leu Pro Gln Leu Glu Gln
1               5


<210>  119
<211>  7
<212>  PRT
<213>  Unknown

<220>
<223>  SARS-COV-2

<400>  119

Val Leu Gln Leu Pro Gln Gly
1               5


<210>  120
<211>  6
<212>  PRT
<213>  Unknown

<220>
<223>  SARS-COV-2

<400>  120

Ala Leu Pro Gln Arg His
1               5


<210>  121
<211>  7
<212>  PRT
<213>  Unknown

<220>
<223>  SARS-COV-2

<400>  121

Ala Leu Gln Leu Glu Glu Glu
1               5

<210>  122
<211>  6
<212>  PRT
<213>  Unknown

<220>
<223>  SARS-COV-2

<400>  122

Leu Pro Leu Gln Leu Gly
1               5

<210>  123
<211>  6
<212>  PRT
<213>  Unknown

<220>
<223>  SARS-COV-2

<400>  123

Val Val Ala Asp Ala Val
1               5

<210>  124
<211>  6
<212>  PRT
<213>  Unknown

<220>
<223>  SARS-COV-2

<400>  124

Arg Leu Ala Leu Gly Gly
1               5

<210>  125
<211>  6
<212>  PRT
<213>  Unknown

<220>
<223>  SARS-COV-2

<400>  125

Leu Gly Gly Leu His Leu
1               5

<210>  126
<211>  7

<212>  PRT
<213>  Unknown

<220>
<223>  SARS-COV-2

<400>  126

Ala Asp Gly Leu Glu Ala Pro
1               5


<210>  127
<211>  7
<212>  PRT
<213>  Unknown

<220>
<223>  SARS-COV-2

<400>  127

Gln Pro Glu Glu Glu Gln Glu
1               5


<210>  128
<211>  9
<212>  PRT
<213>  Unknown

<220>
<223>  SARS-COV-2

<400>  128

Leu Leu Leu Val Ala Ala Gly Leu Glu
1               5


<210>  129
<211>  9
<212>  PRT
<213>  Unknown

<220>
<223>  SARS-COV-2

<400>  129

Leu Leu Leu Val Ala Ala Val Leu Glu
1               5


<210>  130
<211>  9
<212>  PRT
<213>  Unknown

<220>
<223>  SARS-COV-2

<400>  130

Leu Leu Leu Val Ala Val Gly Leu Glu
1               5

<210>  131
<211>  9
<212>  PRT
<213>  Unknown

<220>
<223>  SARS-COV-2

<400>  131

Leu Leu Leu Val Ala Val Val Leu Glu
1               5

<210>  132
<211>  8
<212>  PRT
<213>  Unknown

<220>
<223>  SARS-COV-2

<400>  132

Val Leu Leu Leu Leu Val Ala Ala
1               5

<210>  133
<211>  8
<212>  PRT
<213>  Unknown

<220>
<223>  SARS-COV-2

<400>  133

Val Leu Leu Leu Leu Val Ala Val
1               5

<210>  134
<211>  8
<212>  PRT
<213>  Unknown

<220>
<223>  SARS-COV-2

<400>  134

Val Leu Asp Leu Leu Val Ala Ala
1               5

<210>  135
<211>  8

<212>  PRT
<213>  Unknown

<220>
<223>  SARS-COV-2

<400>  135

Val Leu Asp Leu Leu Val Ala Val
1               5